# EUROPEAN PATENT SPECIFICATION

(11) **EP 0 669 130 B1**
(45) Date of publication and mention of the grant of the patent: **05.11.1997**
(21) Application number: 95300791.1
(22) Date of filing: 08.02.1995
(51) Int. Cl.: A61K 9/20, A23G 3/00

(54) **Tabletting process**
Tablettierungsverfahren
Procédé de fabrication de comprimés

(30) Priority: 24.02.1994 GB 9403484
(43) Date of publication of application: 30.08.1995
(73) Proprietor: CERESTAR HOLDING BV, NL-4550 AA Sas Van Gent (NL)
(72) Inventor: Gonze, Michel Henri Andre, B-1020 Bruxelles (BE); Van der Schueren, Freddy Maurits, B-9300 Alast (BE)
(74) Representative: Wilkinson, Stephen John

(56) References cited:
- EP-A- 0 032 288
- WO-A-88/06845
- FR-A- 2 451 357
- GB-A- 1 187 631

## Description

The present invention relates to a process for making tablets, in particular to a process for making a tablet comprising a sorbitol excipient.

Sorbitol is a hexitol which is made by the reduction of glucose. It is a sweet, crystalline compound which, compared with conventional sweeteners such as glucose and sucrose, has a reduced calorie content and finds applications therefore in dietetic compositions. Sorbitol is also a useful excipient in both pharmaceutical and confectionary tablets and it is with this latter application that the present invention is particularly concerned.

Sorbitol is characterised by its polymorphism, existing in amorphous as well as in a number of crystalline forms. Of the crystalline polymorphs the gamma-crystalline variety is preferred as a tablet excipient but even with this single polymorph it is possible to obtain products having different crystalline matrices depending upon the crystallisation process used.

When employed as an excipient, especially in confectionary products , it is common practice to blend the sorbitol with a flavour eg. a citrus flavour and, if the flavour has been extracted from a natural source, it is convenient to use the flavour in the form of a solution in an appropriate solvent, eg. propylene glycol. We have found however that a liquid flavour and/or solvent can have a deleterious effect on the tablet and the commonly used compression tabletting process in as much as the ejection force required to eject the tablet from the compression equipment becomes too high, with a consequent increase in tablet breakage on the machine.

The consolidation of sorbitol crystals in tablet form occurs by plastic deformation and it appears that the effect of the solvent carrier, or the liquid flavour is to influence adversely the plastic deformation of the crystals. In the absence of the solvent and/or flavour the sorbitol crystals form hard tablets under a low compression force, these tablets being readily expelled from the compression equipment. We have now found however that the adverse effect of a liquid flavour and/or solvent may be mitigated if, instead of using sorbitol made by one crystallisation process as in the usual practice, the tablet excipient comprises a mixture of sorbitols made by two or more different processes of crystallisation.

Patent application WO 88/06845 describes a process for making chewing gum in which at least two types of powdered sorbitol form part of the composition used in the process. The advantages of using the two types of sorbitol, which differ in particle morphology and particle size distribution are said to lie in an optimisation of the texture and processability of the gum. There is in this patent application however no mention of tabletting or of the problems encountered in using liquid flavours and/or carrier solvents in a tabletting process.

According to the present invention therefore a process for the production of a tablet comprising a sorbitol excipient and a liquid flavour or other liquid active ingredient and, optionally, a solvent carrier is characterised in that the sorbitol excipient comprises a mixture of two or more crystalline sorbitols which have been made by different crystallisation processes.

All commercially available sorbitol is made by the reduction of glucose but it is the method whereby the sorbitol so produced is obtained as a solid product that is important with respect of the present invention. There are a number of methods in use namely :-
a) A process which comprises simultaneously mixing and cooling a sorbitol magma in a continuous mixer. This type of process is described in EP 32 288.
b) A process in which molten sorbitol and sorbitol seed are blended and the blend agitated for a period of time under carefully controlled conditions. This type of process and variants thereof are described in GB 1 287 509, GB 1 481 846 and EP 330 352.
c) A process which comprises spray-drying a sorbitol solution. This type of process is described in DD 69 360 and EP 111 209.

The descriptions of the processes and the products produced thereby in these publications are incorporated in the present specification by reference.

Preferably the crystalline sorbitols used in the process of the present invention have been prepared by two or more processes selected from at least two of process types (a), (b) and (c) above.

Sorbitol made by the different processes of crystallisation varies in respect of both particle morphology and particle size distribution. For example, the parameters which define crystal morphology for sorbitol crystallised by two different processes are compared in Table 1 below. The sample A was crystallised by a process of the type described in (a) above ie by use of a continuous mixer as described in EP 32 288. Sample B was prepared by a process of general type (b) above specifically by the process described in USP 2 594 863 in which molten sorbitol having a low water content was fed onto a bed of seed crystals and the seed crystals mixed and rubbed and allowed to age to effect crystallisation. After a suitable period of time the cooled pellets were ground and screened to the desired particle size.

**Table I**

| | A | B |
|---|---|---|
| Melting point | > 98°C | about 97°C |
| Gamma-sorbitol content | about 96 % | about 95 % |
| Specific surface area m²/g | about 0.8 | about 0.3 |
| Appearance | plates in regular, ordered distribution | very fine needles, tightly disposed |

A mixture of sorbitol powders A and B having the above properties has proved to be particularly effective as an excipient in a tablet which comprises a carrier solvent and/or liquid flavour.

Although all combinations of type (a), (b) and (c) can be employed a combination of powders wherein type (a) is an easily compressible sorbitol powder and type (b) is a more rigid crystal which is not easily compressible, is particularly suited for the process of the present invention.

In order to obtain the maximum benefits of the present invention it is preferred to use a mixture of two sorbitols in a weight ratio in the range 90:10 to 10:90 more preferably 80:20 to 20:80. The choice of a ratio within these ranges may be made by reference to the nature of the tablet, the purpose for which it is required and the type of equipment used to make the tablets.

The solvent which may form a part of the tablet made by the process of the invention may be any solvent customarily used as a flavour carrier examples of which include propylene glycol, triethylacetate, benzyl alcohol and triacetin. The amount of liquid, ie. flavour or other active ingredient plus solvent if present is suitably in the range 0.1 to 0.6 % by weight of the tablet.

The process of the present invention will now be further described and illustrated by reference to the following Examples.

In these Examples tablets were produced on Fette P1000 (TM) equipment. The manufacturing process was performed as follows :
- mix Sorbitol powder with the flavour during 2 min.
- add 1.0 % Mg stearate (lubricant)-mix during 2 minutes.
- compress on Fette P1000 (TM)

Pressure used in compression is given in Kilonewtons (kN) and EFM is the required Ejection Force. Both are measured using checkmaster 3 (TM) (Fette).

### Examples 1 - 5

| Example | | Sorbitol Type | Propylene Glycol | Pressure needed to compress the tablet kN | Force needed to eject the tablet* |
|---|---|---|---|---|---|
| 1 | (a) | A | No | 10 | 60 |
| | (b) | A | No | 30 | 120 |
| 2 | (a) | A | Yes | 5 | 390 |
| | (b) | A | Yes | 8 | 700 |
| 3 | (a) | A+B(50/50) | Yes | 13 | 150 |
| | (b) | A+B(50/50) | Yes | 30 | 330 |
| 4 | (a) | A+B(75/25) | Yes | 13 | 140 |
| | (b) | A+B(75/25 | Yes | 25 | 370 |
| 5 | (a) | B* | Yes | 6 | 130 |
| | (b) | B* | Yes | 11 | 380 |

| | | | | | |
|---|---|---|---|---|---|
| B * was a mixture of two sorbitols one being sorbitol B as described earlier in this specification, the other being a sorbitol prepared by another type (b) process. These two sorbitols were present in a ratio of 50 B to 50 other type (b) sorbitol. The "other type (b)" sorbitol was less compressible than sorbitol B. | | | | | |
| * A force greater than 300 confers a significant risk of tablet breakage on the machine. | | | | | |

From the above results it may be seen that whereas when propylene glycol is not present there is only a moderate increase in ejection force when the compression pressure is increased (Examples 1 (a) and (b) ) the addition of propylene glycol causes a dramatic increase in the rate of increase of ejection force for an increase in compression pressure. (Examples 2 (a) and (b) ). When the sorbitols are blended however as in Examples 3 (a) and (b), 4 (a) and (b), 5 (a) and (b) the rate of increase is significantly less.

Improvement depends on the selected blends (A+B) better than B*.

### Examples 6 - 8

Some of the well-known flavour solvents lead to a large ejection force even at low compressing pressure. The ejection force is dramatically lowered by the addition of a second sorbitol powder type in this case type B

| Example | | Sorbitol Type | Solvent | Pressure needed to compress the tablet | Force needed to eject the tablet |
|---|---|---|---|---|---|
| 6 | (a) | A | triethylacetate | 5 | 440 |
| | (b) | A+B(75/25) | | 12.5 | 60 |
| | (c) | A+B(75/25) | | 30 | 95 |
| 7 | (a) | A | benzyl alcohol | 10 | 575 |
| | (b) | A+B(75/25) | | 12.5 | 60 |
| | (c) | A+B(75/25) | | 30 | 100 |
| 8 | (a) | A | triacetin | 10 | 800 |
| | (b) | A+B(75/25) | | 12.5 | 75 |
| | (c) | A+B(75/25) | | 30 | 125 |

### Examples 9-10

The following table shows experiments sorbitol with a finer granulometry.

From the table it can be concluded that finer granulometry gives harder tablets (although this also depends on the morphology of the sorbitol powder). The table shows that combination of fine type A and fine Type B instead of 100% fine type A is particularly advantageous when solvent is used. The ejection force is decreased but tablets of similar hardness are obtained.

| Example | Sorbitol type | Propylene glycol | Pressure needed to compress the tablet | Force needed to eject the tablet | Tablet Hardness kg |
|---|---|---|---|---|---|
| 9 | fine A | No | 5 | 40 | 17 |
| | fine A | Yes | 5 | 220 | 12 |
| 10 | fine A+fine B (50/50) | No | 5 | 40 | 12 |
| | fine A+fine B (50/50) | Yes | 5 | 100 | 13 |

## Claims

1. A process for the production of a tablet comprising a sorbitol excipient and a liquid flavour or other liquid active ingredient and, optionally, a solvent carrier is characterised in that the sorbitol excipient comprises a mixture of two or more crystalline sorbitols which have been made by different crystallisation processes.

2. A process according to claim 1 characterised in that the crystalline sorbitols have been prepared by two or more processes selected from at least two of the following process types :
(a) a process comprising the simultaneous mixing and cooling of sorbitol in a continuous mixer,
(b) a process comprising blending molten sorbitol and sorbitol seed and agitating the blend over a period of time and,
(c) a process which comprises spray-drying a sorbitol solution.

3. A process according to claim 2 characterised in that the mixture comprises a sorbitol made by process (a) and a sorbitol made by process (b).

4. A process according to any one of the preceding claims characterised in that the sorbitol excipient comprises a mixture of two sorbitols in a weight ratio in the range 90:10 to 10:90, preferably 80:20 to 20:80.

5. A process according to any one of the preceding claims characterised in that the solvent carrier is propylene glycol, triethylacetate, benzyl alcohol or triacetin.

6. A process according to any one of the preceding claims characterised in that the amount of liquid present is in the range 0.1 to 0.6 % by weight of the tablet.

## Patentansprüche

1. Verfahren zur Herstellung einer Tablette, die einen Sorbitolträger und einen flüssigen Aromastoff oder einen anderen flüssigen, aktiven Bestandteil und wahlweise einen Lösungsmittelträger umfaßt, dadurch gekennzeichnet, daß der Sorbitolträger eine Mischung aus zwei oder mehreren kristallinen Sorbitolen umfaßt, die durch verschiedene Kristallisationsverfahren hergestellt worden sind.

2. Verfahren nach Anspruch 1, dadurch gekennzeichnet, daß die kristallinen Sorbitole nach einem oder mehreren Verfahren hergestellt worden sind, die ausgewählt sind aus mindestens zwei der folgenden Verfahrentypen:
(a) ein Verfahren, das das gleichzeitige Mischen und Kühlen von Sorbitol in einem kontinuierlichen Mischer umfaßt,
(b) ein Verfahren, das das Mischen von geschmolzenem Sorbitol und Sorbitolkristallisationskeimen und das Rühren der Mischung für einen Zeitraum umfaßt, und
(c) eine Verfahren, das das Sprühtrocknen einer Sorbitollösung umfaßt.

3. Verfahren nach Anspruch 2, dadurch gekennzeichnet, daß die Mischung ein nach Verfahren (a) hergestelltes Sorbitol und ein nach Verfahren (b) hergestelltes Sorbitol umfaßt.

4. Verfahren nach einem der vorhergehenden Ansprüchen, dadurch gekennzeichnet, daß der Sorbitolträger eine Mischung aus zwei Sorbitolen in einem Gewichtsverhältnis im Bereich von 90:10 bis 10:90, bevorzugt von 80:20 bis 20:80, umfaßt.

5. Verfahren nach einem der vorhergehenden Ansprüchen, dadurch gekennzeichnet, daß der Lösungsmittelträger Propylenglycol, Triethylacetat, Benzylalkohol oder Triacetin ist.

6. Verfahren nach einem der vorhergehenden Ansprüchen, dadurch gekennzeichnet, daß die Menge der vorliegenden Flüssigkeit im Bereich von 0,1 bis 0,6 Gew.-% der Tablette ist.

## Revendications

1. Un procédé pour la fabrication d'un comprimé, comprenant un excipient formé par ou comportant des sorbitols, et une flaveur liquide ou un autre ingrédient actif liquide et, éventuellement, un solvant servant de véhicule, se caractérise en ce que l'excipient formé par des sorbitols ou comportant des sorbitols comprend un mélange de deux ou de plus de deux sorbitols cristallins qui ont été préparés par des procédés différents de cristallisation.

2. Procédé selon la revendication 1, caractérisé en ce que les sorbitols cristallins ont été préparés par deux ou plus de deux procédés choisis parmi au moins deux des types de procédés suivants:
(a) un procédé comprenant le mélangeage et le refroidissement simultanés de sorbitol dans un mélangeur fonctionnant en continu;
(b) un procédé comprenant le mélangeage de sorbitol fondu et de semence de sorbitol, et l'agitation du mélange sur une certaine période de temps; et
(c) un procédé qui comprend le séchage par atomisation d'une solution de sorbitol.

3. Procédé selon la revendication 2, caractérisé en ce que le mélange comprend un sorbitol préparé par le procédé (a) et un sorbitol préparé par le procédé (b).

4. Procédé selon l'une quelconque des revendications précédentes, caractérisé en ce que l'excipient sorbitol comprend un mélange de deux sorbitols selon un rapport pondéral compris entre 90:10 et 10:90, de préférence entre 80:20 et 20:80.

5. Procédé selon l'une quelconque des revendications précédentes, caractérisé en ce que le solvant servant de véhicule est du propylène glycol, du "triéthylacétate" (diéthylacétate d'éthyle), de l'alcool benzylique, ou de la triacétine.

6. Procédé selon l'une quelconque des revendications précédentes, caractérisé en ce que la quantité de liquide présent se situe entre 0,1 et 0,6% du poids du comprimé.
